# EUROPEAN PATENT SPECIFICATION

(11) **EP 1 405 967 B1**
(45) Date of publication and mention of the grant of the patent: **07.12.2011**
(21) Application number: 03021710.3
(22) Date of filing: 25.09.2003
(51) Int. Cl.: E05B 17/00, E05C 3/04, A61L 2/24

(54) **A closing device in particular for a steam-sterilizing machine**
Verschlussvorrichtung insbesondere für eine Dampfsterilisierungmaschine
Dispositif de verrouillage notamment pour un appareil pour stériliser à vapeur

(30) Priority: 01.10.2002 IT MI20022074
(43) Date of publication of application: 07.04.2004
(73) Proprietor: M.O.COM. S.r.L., 20124 Milano (IT)
(72) Inventor: Piazzalunga, Gianfranco, 20080 Casarile (Milano) (IT); Tosi, Daniele Marino, 27018 Vidigulfo (Pavia) (IT)
(74) Representative: Lunati, Vittoriano

(56) References cited:
- US-A- 3 217 661
- US-A- 5 313 738
- US-A1- 2002 083 747
- US-B1- 6 391 258

## Description

The invention relates to a closing device in particular for a steam-sterilizing machine, of the type set out in the preamble of Claim 1.

The device can be used to close any container that is put under pressure during its use and for which a closure of maximum safety is required, such as steam-sterilizing machines employed to sterilize instruments and equipment for dental use.

It is known that particularly in dentist's surgeries sterilizing machines are spread that are provided, within a generally prismatic shell, with various devices among which a small boiler is included for steam-sterilization under pressure of the medical instruments to be used.

The boiler has a substantially cylindrical container and the latter comprises a vessel with an opening or mouth and a door for opening and closing the vessel and in engagement with said mouth.

For locking the door several closing devices of different kinds are presently provided. For example, shaped struts are provided that are integral with the door and, at the closure, penetrate into appropriate seats where they are locked by bolts or the like.

The importance of the closing devices in these machines is well apparent due to the presence of high efforts on the door when the boiler is under pressure and due to the fact that management of the machines can be carried out by staff unable to close the door with sufficient force.

For example patents US 3 217 661 and US 5 313 738 describe closing devices that can be used with a steam-sterilizing machine and that may be very difficult to close.

In fact, on the one side when the boiler is under pressure very high forces are present on the door that tend to open the latter, which forces can reach about 15000 Newtons, for example, and on the other hand all operations are often carried out by female staff, the dentist assistants in a dentist's surgery for example.

A drawback in known closing devices exactly consists in the difficulty of same to ensure a strong and reliable closure while simultaneously offering a simple management in which manual locking efforts are not required.

The same drawback is at least partially overcome by a closing device described in US Patent 6 391 258, which provide a door that can be manually and easily engaged with a vessel and means for approaching the door and the vessel after that the same door and vessel are reciprocally engaged. Nevertheless the described closing device is very slow and uncomfortable because off the presence of the two said different steps of engagement and approaching.

Another drawback of the known art is connected with a non-fully reliable door control during the boiler operation so as to avoid in an absolute manner accidental openings operated by mistake when the boiler is still under pressure. A further drawback is linked to the fact that at all events, in order to obtain a good closure of the door, it is necessary to move the same well close to the vessel mouth.

An inadvertent imprecise approaching may lead to a non-closure of the door. Under this situation the technical task underlying the invention is to conceive a closing device, in particular for a steam-sterilizing machine, capable of substantially obviating the mentioned drawbacks.

Within the scope of this technical task, it is an important aim of the invention to conceive a closing device capable of withstanding very high efforts while being able to carry out locking without any effort being required from the staff in charge.

Another important aim of the invention is to conceive a closing device capable of avoiding any accidental opening due to a wrong command during operation of the boiler.

A still further aim of the invention is to make available a closing device in which the door can reach a perfect closure even in the presence of a relatively imprecise approaching of the door itself to the mouth of the vessel to be closed. The technical task mentioned and the aims specified are achieved by a closing device, in particular for a steam-sterilizing machine, as claimed in the appended Claim 1.

Preferred embodiments are described in the subclaims.

Description of a preferred embodiment of a device in accordance with the invention is now set out hereinafter and illustrated in the accompanying drawings, in which:

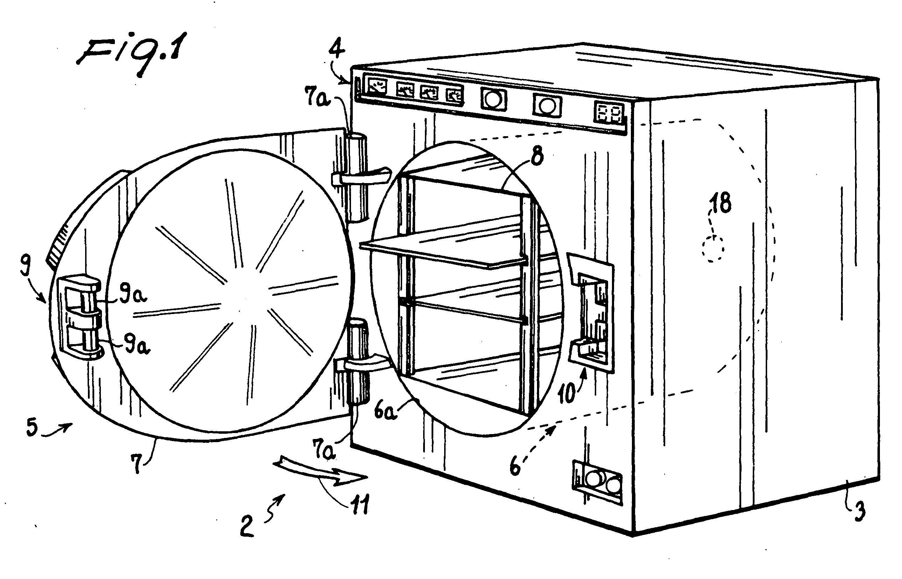

**Fig. 1** shows the device applied to a sterilizing machine for dental use, seen diagrammatically on the whole;

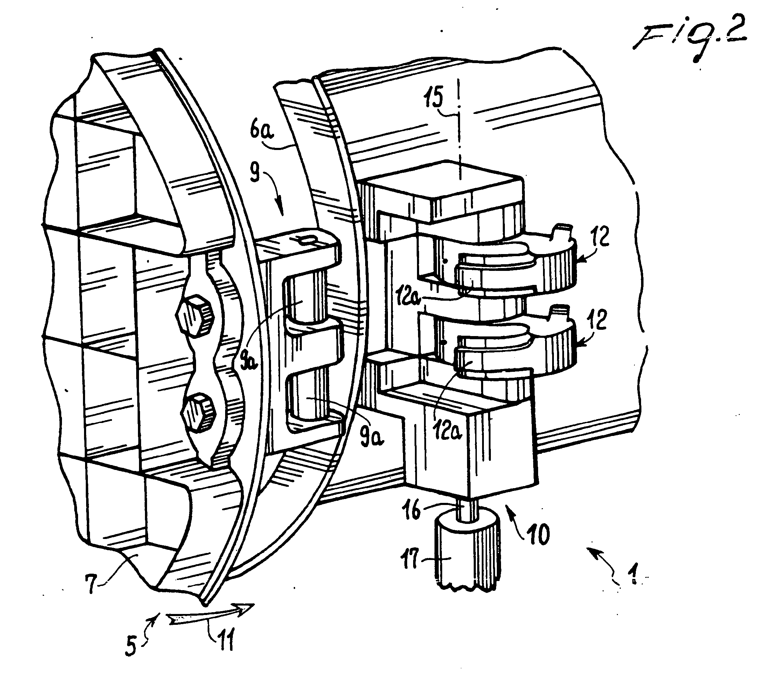

**Fig. 2** is a perspective view of the device taken as a whole;

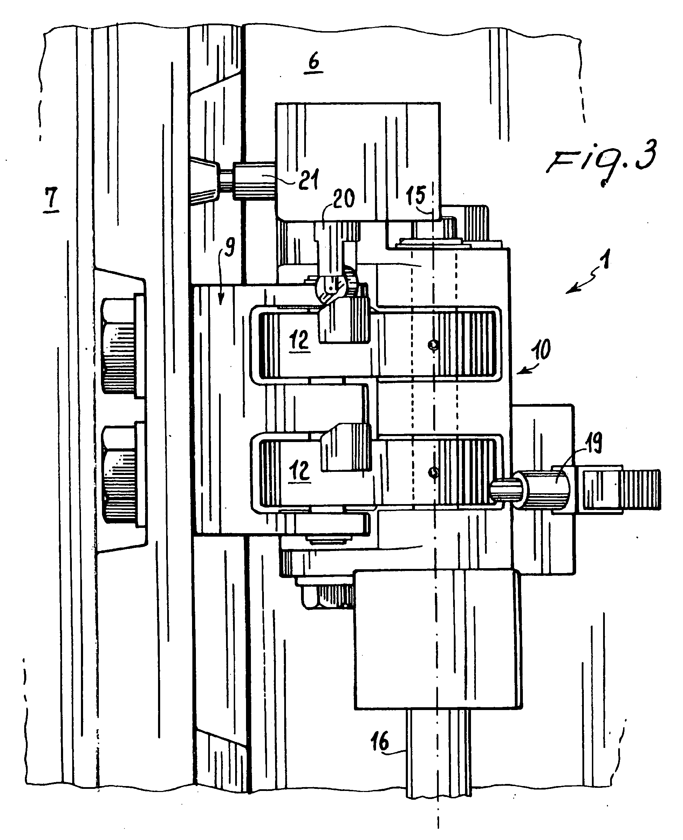

**Fig. 3** is an elevation view of the device in a closed position;

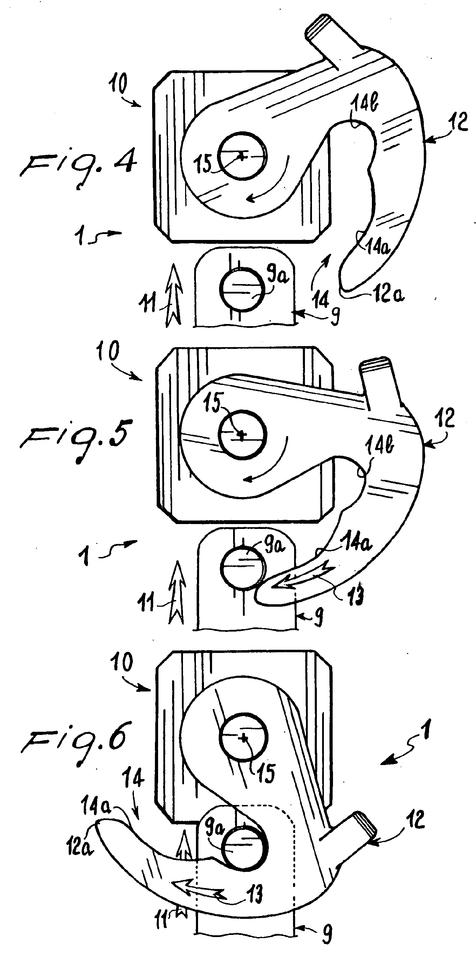

**Figs. 4, 5, 6** are plan views of three operating steps of the device, when from an open position the device reaches a closed position; and

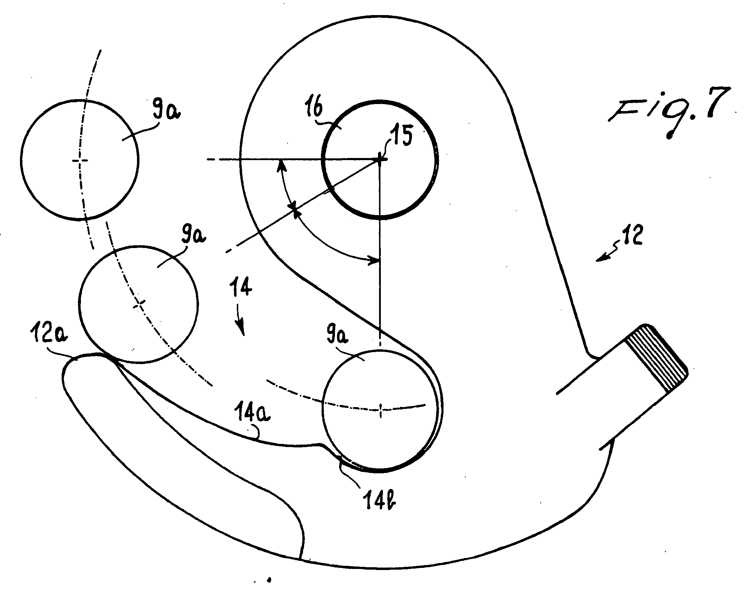

**Fig. 7** diagrammatically shows in a single figure how intervention of the device occurs.

With reference to the drawings, the closing device in accordance with the invention is generally identified by reference numeral **1** and is in particular intended for a steam-sterilizing machine **2** for dentist's surgeries that must sterilize the instruments employed therein.

The machine 2 has a shell **3** and several adjusting controls or commands **4**, in particular for selecting the type of sterilization cycle to be carried out depending on the treated material, and for starting the cycle itself.

Internally of the shell 3 several elements and apparatus of the mechanical, electrical and electronic type are then provided, as well as several sensors and members for conveying, metering and heating the fluid to be vaporised and put under pressure, and to carry out the different sterilization cycles that are made necessary each time.

These different members are described in patents EP 0654274, EP 0742016, EP 0937465 in the name of the same Applicant, for example.

An element of the machine 2 consists of a substantially cylindrical container **5** that is put under pressure and in which sterilization of said instruments takes place. Container 5 comprises an open vessel **6** provided with a mouth **6a**, and a door **7** adapted to close said mouth 6a. Housed internally of vessel 6 is a rack **8** or the like for the objects to be sterilized and the door 7 - that practically embodies an extremity of the cylindrical container 5 - is articulated by means of hinges **7a** on vessel 6 and can be locked by means of the closing device 1.

The closing device 1 comprises grip means **9** and hooking means **10** to be engaged with each other and selectively supported by door 7 and vessel 6; they are relatively movable along an approaching trajectory or first trajectory **11** for mutual approaching imposed by door 7 and in particular by the door hinges 7a. The hooking means 10 comprises at least one holding element **12** and preferably more than one, drivingly movable between an inactive position and a plurality of work positions or engagement positions with the grip means 9, which positions are consecutive to each other.

In particular, each holding element 12 is movable along a locking trajectory or second trajectory **13** substantially transverse to the first one, preferably in the form of an arc of a circle, as better pointed out in the following.

In addition element 12 is provided with a side wall **14** adapted to engage the grip means 9. The side wall 14 has at least one main stretch **14a** extending obliquely between the first trajectory 11 and second trajectory 13.

The side wall 14 consecutively to the main stretch 14a has a final stretch **14b** defining a locking position of the grip means 9. The final stretch 14b is defined by a niche housing the grip means 9 and hollowed out in the respective holding element 12.

The final stretch or niche 14b practically embodies a dead centre or stability point in the presence of efforts tending to move the grip means 9 away from the hooking means 10.

In the specific embodiment shown the hooking means 10 is supported by vessel 6 and the grip means 9 by door 7.

In addition, the hooking means 10 defines a rotation axis **15** for each holding element 12. This rotation axis 15 is substantially perpendicular to the approaching trajectory or first trajectory 11 and to the locking trajectory or second trajectory 13 that is therefore in the form of an arc of a circle.

Each holding element 12 is then defined by a hook extending perpendicular to the rotation axis 15 and provided with a tapered or partly rounded-off free end **12a** and with a side wall 14 having the main stretch 14a shaped with an arched configuration and gradually moving close to the rotation axis 15.

Preferably two holding elements or hooks 12 are present that are spaced apart from each other, and the grip means 9 has two work portions **9a** to be selectively engaged by the two hooks and defined by two spaced coaxial pins or by two spaced portions of a bigger pin. The two hooks and two pins are in a spaced relationship for safety reasons.

Displacement of hooks 12 around the rotation axis 15 is obtained by arranging a stem **16** coaxial with the rotation axis 15 and integral with hooks 12, and a driving member such as a servomotor for example or a reduction motor **17** coaxial with stem 16.

The reduction motor 17, known by itself, or another driving member, preferably has a maximum work power lower than that required for disengagement of the hooking means 10 and grip means 9 from each other in the presence of pressure within the container 5. This maximum work power is obtained by suitably selecting the driving member or reducing or limiting the power of the latter by means of electronic circuits for example, that control operation of the machine 2.

In particular, the reduction motor 17 has a maximum work power lower than that required for disengagement of the work portions 9a from niches 14b formed in the holding elements or hooks 12, in the presence of pressure within the container 5 in a closed position, with the door 7 locked onto the mouth 6a of vessel 6.

As shown, at least one safety sensor is provided which is embodied by a pressure switch **18** adapted to detect pressure inside container 5 or vessel 6 when the same is closed by door 7, and adapted to send signals to the reduction motor 17. Practically the reduction motor 17 is interlocked with the pressure switch 18: operation of the reduction motor is inhibited in the presence of pressure within vessel 6.

Position of the holding elements or hooks 12 is then controlled by a first and a second microswitches **19** and **20** adapted to detect limit positions of hooks 12 and to stop operation of the reduction motor 17.

The microswitches are responsive to the geometric features of hooks 12 and the first microswitch 19 detects the limit position in the opening direction of hooks 12, whereas the second microswitch 20 detects the limit position in the locking direction of door 7.

At least one door-approaching sensor **21** is also provided, intervention of which sensor enables the different operations of device 1 to be carried out and in particular activation of the reduction motor 17 when door 7 is sufficiently close to the mouth 6a of vessel 6.

Operation of the device is as follows.

At the beginning, before intervention of the device, the articles and instruments to be sterilized are introduced into vessel 6, at rack 8. Then door 7 is moved close to mouth 6a at least until activation of the approaching sensor 21.

Under this situation it is possible to intervene by means of controls 4 so as to start a sterilization cycle.

The first operation of the cycle is the automatic and sealing closure of door 7 by intervention of the closing device 1.

For the purpose the driving member embodied by the reduction motor 17 in the example herein shown drives stem 16 in rotation so as to cause rotation of hooks 12. Hooks 12 rotate about the rotation axis 15 causing the forward movement of their free ends 12a that after a rotation of about thirty degrees for example, engage the work portions or pins 9a.

Engagement takes place from the side of the free ends 12a of hooks 12 where the side wall 14 is placed.

Since the free ends 12a are the portions of hooks 12 that are the farthest from the rotation axis 15, the holding elements or hooks 12 are able to be engaged with the work portions or pins 9a even when the latter are relatively spaced from the final position they take when door 7 is perfectly closed.

Practically hooks 12 are able to grasp the grip means 9 of door 7 also when the latter is only disposed close to mouth 6a.

After grasping of pins 9a, hooks 12 still rotate upon command of the reduction motor 17 through sixty degrees for example, making the pins 9a slide on the side wall 14, at its main stretch 14a.

This main stretch has a curved shape gradually approaching the rotation axis 15, and therefore pins 9a are pulled towards vessel 6 and the door 7 is closed on mouth 6a with force.

In the preferred embodiment the main stretch 14a of the side wall 14 tensions pins 9a so that they carry out a door stroke of about two millimetres, from the approached position to the closed position.

Pins 9a by sliding on the side wall 14 internal to hooks 12, reach the final stretch or niche 14b defining the locking position of the grip means 9. When this position has been reached door 7 is sealingly closed, also due to the presence of wide elastic gaskets interposed between door 7 and vessel 6.

The limit stop of the reduction motor 17 is controlled by the second microswitch 20.

The machine 2 can bring container 5 under pressure and steam-sterilize the instruments placed on rack 8.

When the inner pressure is in a steady state condition, at about two bars for example, door 7 must withstand a great effort tending to open it and it is essential that under this situation an accidental opening of door 7 be impossible, even in the presence of a possible wrong opening command.

In this case intervention of the safety sensor or pressure switch 18 occurs, which sensor prevents rotation of the reduction motor 17 to an open position when it detects the presence of a non marginal pressure in container 5.

Door 7 can therefore be opened only in a safe manner and a wrong command is ineffective.

A further security is represented by the presence of the reduction motor 17 itself that, due to its operating features, prevents any possible attempt to manually move hooks 12: the same can be rotated around the rotation axis 15 only through intervention of the reduction motor.

A further important security is obtained by niche 14b; thanks to this niche the efforts on door 7 do not cause sliding or rotation of hooks 12 to an open position, since the niche itself defines a stable dead centre by virtue of which the efforts exerted on the door tend to close the door itself.

Stability is lost if efforts are exerted which are transverse to those exerted by pressure inside the container and tending to rotate hooks 12. But these transverse efforts must grow as the active pressures on door 7 increase, which pressures would tend to increasingly push pins 9a into niche 14b.

Conveniently, the driving member embodied by the reduction motor 17 can be calibrated or reduced in such a manner that it cannot operate overcoming of said stability point in the presence of a pressure acting on door 7 from the inside. Therefore, even in case of failure of the pressure switch 18 and of a concurrent wrong command for opening the door, the reduction motor 17 is unable to open the door if the latter is under pressure.

When the sterilization cycle has been completed and the electronic control circuits contemplate opening of door 7, the pressure switch 18 detects the absence of inner pressure and enables activation of the reduction motor 17 with rotation in the direction of disengagement of the hooking means 10 from the grip means 9.

Hooks 12 can rotate because the mechanical lock exerted by niche 14b is no longer present to an important degree due to the absence of pressure on door 7. Rotation of hooks 12 goes on through about ninety degrees until the geometrical features of the hooks interfere with the first microswitch 19 that stops rotation. The door can be now rotated to its opened condition to enable extraction of the sterilized articles.

The invention achieves important advantages.

In fact the closing device as conceived is reliable in terms of solidity and safety, because various types of elements and controls are provided that make it impossible to accidentally open the door.

The presence of a mechanical security is also to be underlined, which mechanical security is represented by the niches, the efficiency of which grows on increasing of the efforts at stake. Under conditions of high efforts the action of the reduction motor itself is also inhibited.

The closing device is also able to avoid any physical closing effort because the door can also be merely approached before closure: the device itself does the necessary to bring the door to a position at which it closely approaches the vessel.

The invention is susceptible of many modifications and variations.

For example, the grip means can be supported by the vessel and the hooking means by the door. The hooks can be of any number and they can also be replaced by other elements, inclined planes for example that are caused to slide so as to engage and tension the grip means.

## Claims

1. A steam-sterilizing machine comprising a closing device, said machine comprising a vessel (6), a mouth (6a) for vessel loading and unloading, and a door (7) adapted to close said mouth (6a), and said closing device comprising grip means (9) and hooking means (10) to be engaged with each other and selectively supported by said door (7) and said vessel (6) and relatively movable along an approaching trajectory (11), said hooking means (10) comprising at least one holding element (12) drivingly movable along a locking trajectory (13) substantially transverse to said approaching trajectory (11) and provided with at least one side wall (14) for engagement with said grip means (9) having a main stretch (14a) extending at least partly obliquely between said approaching trajectory (11) and locking trajectory (13), wherein a rotation axis (15) for said at least one holding element 12 is provided which is substantially perpendicular to said approaching trajectory (11) and said locking trajectory (13), wherein said side wall (14) has said main stretch (14a) of an arched shape and gradually moving close to said rotation axis (15) and wherein said hooking means (10) comprises a stem (16) coaxial with said rotation axis (15) and integral with said at least one holding element (12), and **characterised in that** a motor operated driving member (17) is provided coaxial with the stem for control of said stem (16).

2. A device as claimed in Claim 1, wherein said side wall (14) has a final stretch (14b) comprising a hollow housing niche defining a stability point for said grip means (9) in the presence of efforts tending to move said grip means (9) away from said hooking means (10).

3. A device as claimed in Claim 1, wherein said at least one holding element (12) is substantially a hook.

4. A device as claimed in Claim 3, wherein two holding elements (12) are provided that are substantially defined by two hooks spaced apart from each other, and wherein said grip means (9) has two work portions (9a) to be selectively engaged by said two hooks (12) and substantially defined by spaced pins.

5. A device as claimed in Claim 1, and wherein said driving member (17) has a maximum work power lower than that required for disengaging said hooking means (10) and grip means (9) from each other in the presence of pressure in said vessel (6).

6. A device as claimed in Claim 2, wherein said driving member (17) has a maximum work power lower than that required for disengaging said grip means (9) from said niche (14b), in the presence of pressure within said vessel (6).

7. A device as claimed in Claim 1, wherein at least one safety sensor (18) is provided which is adapted to detect the inner pressure of said vessel (6), and wherein said driving member (17) is interlocked with said safety sensor (18).

8. A device as claimed in Claim 1, wherein microswitches (19, 20) are provided which are adapted to detect limit positions of said at least one holding element (12), and wherein said driving member (17) is interlocked with said microswitches (19, 20).

## Patentansprüche

1. Dampfsterilisierungsmaschine, umfassend eine Verschlussvorrichtung, wobei besagte Maschine einen Behälter (6), eine Öffnung (6a) zum Beladen und Entladen des besagten Behälters (6) und eine Klappe (7) umfasst, die geeignet ist, die besagte Öffnung (6a) zu verschließen, und wobei besagte Verschlussvorrichtung Greifmittel (9) und Verriegelungsmittel (10) umfasst, die einander in Anspruch nehmen und selektiv von besagter Klappe (7) und von besagtem Behälter (6) getragen werden und im relativen Sinn längs einer Annäherungsbahn (11) beweglich sind, wobei besagte Verriegelungsmittel (10) mindestens ein auf Befehl längs einer Blockierungsbahn (13) bewegliches Greifelement (12) umfassen, das im Wesentlichen quer zu der besagten Annäherungsbahn (11) liegt und mindestens eine Einsatzkante (14) zu besagten Greifmitteln (9) aufweist, die eine Hauptstrecke (14a) besitzt und sich mindestens teilweise quer zwischen besagter Annäherungsbahn (11) und besagter Blockierungsbahn (13) entwickelt, wobei eine Rotationsachse (15) für besagtes mindestens ein Greifelement (12) vorgesehen ist, das im Wesentlichen senkrecht zu der besagten Annäherungsbahn (11) und der besagten Blockierungsbahn (13) liegt, und wobei besagter Hauptabschnitt (14a) der besagten Einsatzkante (14) bogenförmig ist und sich allmählich der besagten Rotationsachse (15) nähert, und wobei besagte Verriegelungsmittel (10) eine Welle (16) umfassen, die zur besagten Rotationsachse (15) koaxial ist und mit besagtem mindestens einem Greifelement (12) fest verbunden ist, **gekennzeichnet durch** die Tatsache, dass ein bewegendes Organ (17) vorgesehen ist, das für die Steuerung der besagten Welle (16) von einem Motor angetrieben wird und koaxial zu der besagten Welle (16) liegt.

2. Vorrichtung nach Anspruch 1, bei der besagte Kante (14) einen Endabschnitt (14b) präsentiert, der eine ausgehöhlte Aufnahmenische umfasst und einen Stabilitätspunkt für besagte Greifmittel (9) bei Kräften definiert, die dazu tendieren, besagte Greifmittel (9) von besagten Verriegelungsmitteln (10) zu entfernen.

3. Vorrichtung nach Anspruch 1, bei der besagtes mindestens ein Greifelement (12) im Wesentlichen ein Haken ist.

4. Vorrichtung nach Anspruch 3, bei der zwei Greifelemente (12) vorgesehen sind, die im Wesentlichen von zwei in einem gewissen Abstand befindlichen Haken definiert werden, und bei der besagte Greifmittel (9) Arbeitsbereiche (9a) aufweisen, die selektiv von besagten zwei Haken (12) in Anspruch genommen werden können und im Wesentlichen von zwei untereinander in einem gewissen Abstand befindlichen Stiften definiert werden.

5. Vorrichtung nach Anspruch 1, bei der besagtes bewegendes Organ (17) eine maximale Arbeitsleistung aufweist, die niedriger ist als die, die notwendig ist, um besagte Verriegelungsmittel (10) und besagte Greifmittel (9) bei Druck in besagtem Behälter (6) voneinander zu lösen.

6. Vorrichtung nach Anspruch 2, bei der besagtes bewegendes Organ (17) eine maximale Arbeitsleistung aufweist, die niedriger ist als die, die notwendig ist, um besagte Greifmittel (9) bei Druck im Inneren des besagten Behälters (6) aus besagter Nische (14b) zu lösen.

7. Vorrichtung nach Anspruch 1, bei der mindestens ein Sicherheitssensor (18) vorgesehen ist, der geeignet ist, den Druck im Inneren des besagten Behälters (6) festzustellen, und bei der besagtes bewegendes Organ (17) dem besagten Sicherheitssensor (18) unterstellt ist.

8. Vorrichtung nach Anspruch 1, bei der Mikroschalter (19, 20) vorgesehen sind, die geeignet sind, die Anschlagpositionen von besagtem mindestens einem Greifelement (12) festzustellen, und bei der besagtes bewegliches Organ (17) den besagten Mikroschaltern (19, 20) unterstellt sind.

## Revendications

1. Appareil pour stériliser à vapeur, comprenant un dispositif de verrouillage, ledit appareil comportant un récipient (6), un orifice de chargement et de décharge dudit récipient (6), et une porte (7) apte à fermer ledit orifice (6a), et ledit dispositif de verrouillage comprenant des moyens de prise (9) et des moyens d'accrochage (10) susceptibles de s'engager réciproquement et sélectivement, supportés par ladite porte (7) et par ledit récipient (6) et mobiles en sens relatif le long d'une trajectoire de rapprochement (11), lesdits moyens d'accrochage (10) comprenant au moins un élément de prise (12) mobile sur commande le long d'une trajectoire de blocage (13) sensiblement transversale à ladite trajectoire de rapprochement (11) et présentant au moins un bord (14) d'engagement avec lesdits moyens de prise (9) ayant une partie principale (14a) s'étendant au moins partiellement obliquement entre ladite trajectoire de rapprochement (11) et ladite trajectoire de blocage (13), dans lequel on prévoit un axe de rotation (15) pour ledit au moins un élément de prise (12) sensiblement perpendiculaire à ladite trajectoire de rapprochement (11) et à ladite trajectoire de blocage (13), et dans lequel ledit bord (14) a ladite partie principale (14a) arquée et se rapprochant graduellement dudit axe de rotation (15) et dans lequel lesdits moyens d'accrochage (10) comprennent un arbre (16) coaxial par rapport audit axe de rotation (15) et solidaire dudit au moins un élément de prise (12), **caractérisé en ce qu'**on prévoit un organe moteur (17) entraîné par un moteur et coaxial par rapport audit arbre (16), pour commander ledit arbre (16).

2. Dispositif selon la revendication 1, dans lequel dit rebord (14) présente une partie finale (14b) comprenant une niche de logement creuse et définissant un point de stabilité pour lesdits moyens de prise (9) en présence d'efforts tendant à éloigner lesdits moyens de prise (9) desdits moyens d'accrochage (10).

3. Dispositif selon la revendication 1, dans lequel ledit au moins un élément de prise (12) est essentiellement un crochet.

4. Dispositif selon la revendication 3, dans lequel on prévoit deux éléments de prise (12) essentiellement définis par deux crochets espacés entre eux, et dans lequel lesdits moyens de prise (9) présentent deux portions de travail (9a) qui peuvent être engagées sélectivement par lesdits deux crochets (12) et essentiellement définies par des pivots réciproquement espacés.

5. Dispositif selon la revendication 1, dans lequel ledit organe moteur (17) présente une puissance maximum de travail plus basse que celle nécessaire pour dégager les uns des autres lesdits moyens d'accrochage (10) et de prise (9) en présence de pression dans ledit récipient (6).

6. Dispositif selon la revendication 2, dans lequel ledit organe moteur (17) présente une puissance maximum de travail plus basse que celle nécessaire pour dégager lesdits moyens de prise (9) de ladite niche (14b), en présence de pression à l'intérieur dudit récipient (6).

7. Dispositif selon la revendication 1, dans lequel on prévoit au moins un capteur de sécurité (18) apte à détecter la pression à l'intérieur dudit récipient (6), et dans lequel ledit organe moteur (17) est assujetti audit capteur de sécurité (18).

8. Dispositif selon la revendication 1, dans lequel on prévoit des microinterrupteurs (19, 20) destinés à détecter des positions de fin de course dudit au moins un élément de prise (12), et dans lequel ledit organe moteur (17) est assujetti auxdits microinterrupteurs (19, 20).
